# EUROPEAN PATENT SPECIFICATION

(11) **EP 1 765 309 B1**
(45) Date of publication and mention of the grant of the patent: **05.08.2009**
(21) Application number: 05752156.9
(22) Date of filing: 08.06.2005
(51) Int. Cl.: A61K 9/70, A61K 9/10, A24B 15/16, A61K 31/465

(54) **SMOKELESS TOABACCO PRODUCT**
RAUCHLOSES TABAKPRODUKT
PRODUIT DE TABAC SANS FUMEE

(30) Priority: 02.07.2004 SE 0401721; 02.07.2004 US 584494 P
(43) Date of publication of application: 28.03.2007
(73) Proprietor: Radi Medical Biodegradable AB, 754 50 Uppsala (SE)
(72) Inventor: ENGSTRÖM, Thomas, S-752 36 UPPSALA (SE); MATHISEN, Torbjörn, 125 42 ÄLVSJÖ (SE)
(74) Representative: Holmberg, Nils Anders Patrik
(86) International application number: PCT/SE2005/000864
(87) International publication number: WO 2006/004480

(56) References cited:
- WO-A1-88/08298
- WO-A1-20/04060353
- WO-A2-00/13680
- DE-A1- 10 157 124
- US-A- 5 760 049
- US-A- 5 783 207
- US-A1- 2005 136 112

## Description

### Field of the Invention

The present invention relates generally to a smokeless tobacco product in the form of a completely dissolvable vehicle comprising concentrated tobacco extract contained in a dissolvable matrix.

### Background of the Invention

Smokeless tobacco, e.g. so called "snuff", is widely used as substitute for cigarettes, cigars and other forms of smoking-tobacco as it poses comparatively less health risks to the user. Many people do also use a smokeless alternative during a transition period to reduce the acute abstinence from nicotine that may results from a sudden refraining from cigarettes.

To make such tobacco more user-friendly, it is known to confine the tobacco in a small non-dissolvable, permeable pouch, which is kept in the mouth until the tobacco flavours have been extracted from the tobacco and which is then removed. It is, however, also well-known that many people find the use of such tobacco pouches little appealing or even repulsive, mainly because of the necessity to remove the saliva soaked and soggy tobacco pouch from the mouth and then dispose the same. The discomfort is, of course, even more pronounced if the smokeless tobacco is used in a public environment, such as meeting rooms, restaurants or other restricted areas, where it can be difficult or even impossible to dispose the used tobacco pouch in a discreet and hygienic way. Needless to say, these people are even less appealed by smokeless tobacco that is not contained in such a pouch.

For the mucosal administration of drugs such as nicotine, drug delivering vehicles in the forms of membranes or patches are known. When such membranes or patches are used for the oral administration of drugs, they do not, however, create a three-dimensional sensation in the user's mouth. It is the present inventors' belief that such a three-dimensional sensation is crucial for some people's acceptance of smokeless tobacco products.

A more appealing smokeless tobacco product is therefore needed.

### Summary of the Invention

Today smokeless tobacco is sold in the form of non-dissolvable tobacco-filled permeable pouches, which a user keeps in his or her mouth until the flavours have been extracted from the tobacco and which the user then removes with his/her fingers, something that many people find unappealing.

The general object of the present invention is to provide a more appealing administration form for smokeless tobacco.

Another object of the invention is to provide a vehicle that, in the mouth of a user, releases flavours coming from tobacco extracts as well as other substances, which vehicle provides for a controlled release and thereby intake of these flavours and substances.

A further object is to provide a three-dimensional vehicle which in the mouth of a user creates a three-dimensional sensation.

These objects are achieved with a dissolvable vehicle comprising a water-soluble polymer matrix in which tobacco extract has been incorporated, wherein the dissolvable vehicle is a multilayer vehicle and the dissolvable polymeric matrix constitutes at least one of the several layers. The tobacco extract has been freed from any type of insoluble residue, and the whole vehicle will therefore dissolve within the mouth of a user. The necessity of an unpleasant and discomfortable removal of a saliva-soaked tobacco pouch is thereby eliminated. In one embodiment of the invention, the dissolvable vehicle is a three-dimensional vehicle, which in the mouth of a user creates a three-dimensional sensation.

According to the present invention, the dissolvable three-dimensional vehicle has a multilayer structure, with at least one layer comprising a water-soluble polymer matrix in which concentrated tobacco extract has been incorporated. Another layer of the three-dimensional vehicle may comprise different kinds of flavours, additives, and other drugs such as nicotine. In one embodiment of the invention, a multilayered three-dimensional vehicle is adapted to dissolve layer by layer, thereby providing for a controlled release and subsequent intake of the drugs and flavours contained in the respective layers.

### Brief Description of the Drawings

Fig. 1 is a schematic illustration of a first embodiment.
Fig. 2 is a schematic illustration of a second embodiment of the present invention.
Fig. 3 illustrates schematically the dimensions of a vehicle according to one embodiment of the present invention.

### Detailed Description of Preferred Embodiments

The present invention relates to a smokeless tobacco product in the form of a dissolvable vehicle comprising a water-soluble polymer matrix containing concentrated tobacco extract, wherein the dissolvable vehicle is a multilayer vehicle and the dissolvable polymeric matrix constitutes at least one of the several layers. The polymer matrix is made from any type of water-soluble polymer, including both synthetic and naturally occurring polymeric substances. The matrix is formulated to optimize the leaching process of the various flavour substances found in tobacco extracts. In one embodiment of the invention, the dissolvable vehicle is a body with significant extensions in all three dimensions, such that the dissolvable vehicle at the start of the dissolution process creates a three-dimensional feeling in a user's mouth.

Examples of suitable water-soluble polymers are various proteins such as collagen or fibrin, and various carbohydrates like alginates, guar gum, agarose, as well as various types of glucosamino-glucanes and cellulose. It can be noted that most of these carbohydrates can be chemically substituted to significantly alter their properties. Various types of polymeric alcohols, such as polyethylene glycols, polypropylene glycols, and polyvinyl alcohols, can be used to formulate the water-soluble matrix as well as polypyrollidone, and various polymeric acrylic acids and ionomers. Several of the alternatives listed above can be cross-linked in various degrees to achieve alterations in their dissolution properties. It is further possible to incorporate various types of non-ionic, cationic and/or anionic substituents in the polymer matrix to yield satisfying dissolution properties, and also to accelerate or retard the leaching process of a specific tobacco flavour.

As already indicated above, the dissolvable vehicle is a multilayer vehicle. One of the layers, preferably the outermost layer, can comprise a component with a certain degree of mucoadhesive properties. An example of such a mucoadhesive substance is chitosan. A mucoadhesive layer would adhere to the mucosa in the mouth of a user, and thereby act as a fixation for the vehicle. Such a mucoadhesive substance could also promote the penetration of tobacco flavours into the mucosa.

As used herein, the terms "soluble" or "dissolvable" are meant to encompass all types of disintegrating, dissolving, resorbing, absorbing and eroding processes that can occur in a vehicle that in a user's mouth dissolves in such a way that a removal from the mouth and a subsequent disposal of a vehicle rest by hand are not necessary after the tobacco flavours have been extracted. The different substances mentioned above are therefore merely illustrative examples of various ingredients that could be used to formulate a self-disintegrating matrix that constitute a base for a dissolvable vehicle for a smokeless tobacco product.

The smokeless tobacco product of the present invention is based on concentrated tobacco extract, which can be extracted by several methods well-known in the art. Tobacco extract is perhaps most easily prepared by treating tobacco with water, or water containing a small amount of an organic solvent. The preferred temperature for extraction is room temperature, but any temperature in the range of 10 °C to 60 °C will work. Also the pH-value and ionic strength can be adjusted to depress or promote extraction of certain tobacco substances. Supercritical carbon dioxide has, for example, been successfully employed to produce tobacco extracts that are diminished in nicotine.

The tobacco extract may include extracts from all original substances of the tobacco plant or a subset thereof, including one or many original substance(s).
In addition, and within the scope of the present invention as it is defined by the claims, the tobacco extract may be a synthetically prepared substance having essentially equivalent characteristics as the corresponding extract from the tobacco plant. Naturally, the extract may include both one or many original (natural) substance(s) and one or many synthetic substance(s).
According to a preferred embodiment the tobacco extract is nicotine or synthetically prepared nicotine, being the only substance or used in combination with other substances.

Below exemplifying embodiments of a vehicle for a smokeless tobacco product are described with reference to the accompanying drawings.

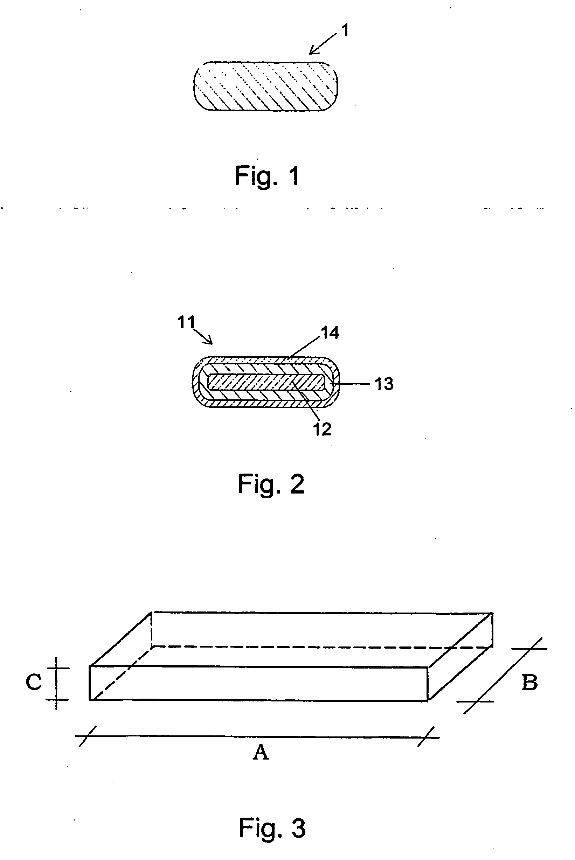

Fig. 1 illustrates a first and basic embodiment of a smokeless tobacco product. The tobacco product comprises a vehicle 1, which is made from a water-soluble polymeric matrix in which tobacco extract has been included. The polymeric matrix can be made from any of the polymers listed above. One way to formulate such a tobacco-containing polymeric matrix would be to dissolve one or several suitable polymers in a water-solution of tobacco extracts. The solution is then dried to a suitable matrix form. During the drying process the polymers can optionally be cross-linked to alter their dissolution properties. A polymer material to be used in the present invention should preferably be soft; it could, for example, have a compression modulus less than 0.1 MPa. The polymer material can have a dissolution time ranging from about 5 minutes to about 60 minutes, and preferably from approximately 10 minutes to approximately 30 minutes.

In Fig. 2, an embodiment of a vehicle 11 for a smokeless tobacco product according to the present invention is shown. The vehicle 11 is a multi-layer vehicle that comprises an inner layer 12, an intermediate layer 13, and an outer layer 14. Here, the inner layer 12 comprises a water-soluble polymeric matrix in which tobacco extract has been included. The matrix can be formulated in the same way as suggested in conjunction with Fig. 1, or the polymeric matrix can be in the form of a porous or fibrous matrix in which dry tobacco extract or water-dissolved tobacco extract is incorporated. The intermediate layer 13 can then serve as a cover or seal that encapsulates the tobacco extract inside the polymeric matrix. The intermediate layer 13 can further comprise a swelling substance that absorbs saliva and thereby increases the volume of the vehicle 11. Any type of cross-linked carbohydrates could be used as a swelling substance. The outer layer 14 can comprise an adhesive, such as chitosan, which adhere to the mucosa in a user's mouth, and thereby fixates the vehicle 11 inside the user's mouth. The outer layer 14 can also comprise a substance that facilitates the penetration of the flavours and other drugs and substances into the mucosa. The structure of the vehicle 11 is only exemplifying, and it should in particular be recognized that more layers could be added, and that the contents of each layer could be altered such that, for example, the swelling substance is in the innermost layer, while the tobacco extract is contained in an intermediate layer.

The different layers of the multilayer vehicle can be based on the same polymer matrix, the only difference being that different additives, such as drugs and flavours, are included in the different layers, with at least one layer containing the tobacco extract. Another possibility is to prepare a special polymeric matrix for each layer to thereby make it easy to control the dissolution of the layer and the release of substances therefrom. It is also conceivable to apply one layer, e.g. the outermost layer, in the form of a dragée, which further can comprise a substance which renders this layer a special effect on the user.

An advantage of having a multilayer vehicle is that the separate layers can be adapted to dissolve sequentially in a user's mouth. In other words, on outer layer is essentially disintegrated before an inner layer starts to dissolve. It is thereby easy to control the affect that the present vehicle has on a user. As an example, the outermost layer could include a high concentration of nicotine, as the desire for nicotine presumably is at the highest just after the user has put the vehicle in his or her mouth, while a more interior layer could include a lower concentration of nicotine, when the user's urge for nicotine has been almost satisfied. The total intake of nicotine could thereby be reduced.

The present invention is not restricted to any particular dimensions of a vehicle for a smokeless tobacco product. The vehicle can, for example, be in the form of an essentially two-dimensional structure such as a thin sheet or patch. It is, however, the present inventors' belief that for some people it is important that a smokeless tobacco product for oral administration is capable of creating a three-dimensional sensation in the mouth of a user. As used herein, the term "three-dimensional" is defined as a dimensional relationship that creates a three-dimensional sensation in a user's mouth.

As an example, most of the plasters and patches known in the prior art do not fulfil this criterion. In one embodiment of the present invention, the vehicle for the tobacco extract has therefore a first dimension (e.g. a length), a second dimension (e.g. a width) and a third dimensions (e.g. a height), and is **characterized in that** none of the three dimensions is negligible in comparison with one of the other two dimensions. When such a vehicle is placed in a user's mouth, the user will experience a sensation that the vehicle occupies space in all three dimensions. When, for example, placed between a lip and teeth or gums, the vehicle would slightly stretch the user's lip to provide essentially the same sensation that is created by a conventional tobacco pouch.

If a vehicle for the mucosal administration of tobacco extracts has a length denoted by A, a width denoted by B, and a height denoted by C, and if A is larger than (or equal to) B, then C should be at least one tenth of A (i.e. C/A ≥ 1/10), preferably C/A ≥ 1/7. A suitable interval for C/A could be 1/8 ≤ C/A ≤ 1/2. Fig. 3 shows a schematic view of the vehicle according to the present invention.

In one embodiment A is about 30 mm, B is about 15 mm, and C is about 5 mm, such that C/A ≈ 1/6. Preferably, a vehicle according to the present invention is pillow-shaped, and provided with rounded corners and/or edges, as can be seen in Fig. 1 or Fig. 2.

Here it can be noted that the three-dimensional sensation referred to above relates to a three-dimensional sensation created when the vehicle has been placed in the mouth of a user. In other words, the vehicle can be essentially two-dimensional before it is placed in the user's mouth. This would in particular apply to a vehicle comprising a swelling substance. Such a vehicle would then perhaps not fulfil the above criteria for A, B and C before it has been placed in the mouth, but would fulfil these criteria shortly after it has come into contact with salvia. Also such vehicles are considered to be three-dimensional vehicles.

Although the present invention has been described with reference to specific embodiments, also shown in the appended drawings, it will be apparent for those skilled in the art that many variations and modifications can be done within the scope of the invention as described in the specification and defined with reference to the claims below. It should in particular be recognized that a vehicle containing tobacco extract can comprise a large variety of different drugs. One example of such a drug is nicotine, which can be included in different, selectable concentrations in different versions of the smokeless tobacco product of the present invention. The nicotine concentration could, for example, range from a high concentration to zero, such that people, e.g. inveterate smokers, gradually can reduce their daily intake and finally get rid of their nicotine addiction. The smokeless tobacco product could also be flavoured with extra flavours, such as liquorice, menthol, spearmint or peppermint. It is also possible to add vitamins and minerals to create a healthier product. More dedicated drugs could also be added. Examples of such drugs would be flour or a suitable teeth-whitening substance, or a substance that cures halitosis, or some other type of nature-cure drug.

## Claims

1. Smokeless tobacco product in the form of a dissolvable vehicle (1; 11) for oral administration, said vehicle being **characterized in that** the vehicle comprises a dissolvable polymeric matrix in which tobacco extract, or a synthetic equivalent thereof, is included, and that said dissolvable vehicle is a multilayer vehicle (11) and that said dissolvable polymeric matrix constitutes at least one of the several layers (12, 13, 14).

2. Smokeless tobacco product according to claim 1, **characterized in that** the tobacco extract is dried tobacco extract.

3. Smokeless tobacco product according to claim 1 or 2, **characterized in that** the outermost layer comprises a mucoadhesive substance.

4. Smokeless tobacco product according to anyone of claims 1 to 3, **characterized in that** at least one layer comprises a substance that swells upon contact with saliva.

5. Smokeless tobacco product according to anyone of claims 1 to 4, **characterized in that** the multilayer vehicle essentially dissolves layer by layer in a user's mouth.

6. Smokeless tobacco product according to anyone of the previous claims, **characterized in that** the vehicle has an essentially two-dimensional shape.

7. Smokeless tobacco product according to anyone of claims 1 to 5, **characterized in** the vehicle is, or when placed in a user's mouth swells to, a three-dimensional vehicle, which has a first dimension A, a second dimension B and a third dimension C, with A ≥ B ≥ C and C/A ≥ 1/10.

8. Smokeless tobacco product according to claim 7, **characterized in that** 1/8 ≤ C/A ≤ 2/3.

9. Smokeless tobacco product according to claim 8, **characterized in that** 1/7 ≤ C/A ≤ 1/2.

10. Smokeless tobacco product according to at anyone of the previous claims, **characterized in that** said vehicle has a compression modulus less than 0.1 MPa.

11. Smokeless tobacco product according to anyone of the previous claims, **characterized in that** said vehicle has a dissolution time in the interval of 5 to 60 minutes, preferably in the interval of 10 to 30 minutes.

12. Smokeless tobacco product according to anyone of the previous claims, **characterized in that** said extract is free from nicotine.

13. Smokeless tobacco product according to anyone of claims 1-11, **characterized in that** said extract is nicotine or synthetically prepared nicotine.

## Patentansprüche

1. Rauchloses Tabakprodukt in Form eines löslichen Vehikels (1; 11) zur oralen Verabreichung, wobei das Vehikel **dadurch gekennzeichnet ist, daß** das Vehikel eine lösliche polymere Matrix umfaßt, die Tabakextrakt oder ein synthetisches Äquivalent davon enthält, und wobei es sich bei dem löslichen Vehikel um ein mehrschichtiges Vehikel (11) handelt und die lösliche polymere Matrix wenigstens eine der mehreren Schichten (12, 13, 14) bildet.

2. Rauchloses Tabakprodukt nach Anspruch 1, **dadurch gekennzeichnet, daß** es sich bei dem Tabakextrakt um getrockneten Tabakextrakt handelt.

3. Rauchloses Tabakprodukt nach Anspruch 1 oder 2, **dadurch gekennzeichnet, daß** die äußerste Schicht eine mucoadhäsive Substanz enthält.

4. Rauchloses Tabakprodukt nach einem der Ansprüche 1 bis 3, **dadurch gekennzeichnet, daß** wenigstens eine Schicht eine Substanz enthält, die, wenn sie mit Speichel in Kontakt kommt, aufquillt.

5. Rauchloses Tabakprodukt nach einem der Ansprüche 1 bis 4, **dadurch gekennzeichnet, daß** sich das mehrschichtige Vehikel im wesentlichen Schicht für Schicht im Mund eines Anwenders auflöst.

6. Rauchloses Tabakprodukt nach einem der vorherigen Ansprüche, **dadurch gekennzeichnet, daß** das Vehikel eine im wesentlichen zweidimensionale Form aufweist.

7. Rauchloses Tabakprodukt nach einem der Ansprüche 1 bis 5, **dadurch gekennzeichnet, daß** es sich bei dem Vehikel um ein dreidimensionales Vehikel handelt bzw. daß das Vehikel, wenn es in den Mund eines Anwenders gegeben wird, zu einem dreidimensionalen Vehikel aufquillt, welches eine erste Dimension A, eine zweite Dimension B und eine dritte Dimension C hat, wobei A ≥ B ≥ C und C/A ≥ 1/10.

8. Rauchloses Tabakprodukt nach Anspruch 7, **dadurch gekennzeichnet, daß** 1/8 ≤ C/A ≤ 2/3.

9. Rauchloses Tabakprodukt nach Anspruch 8, **dadurch gekennzeichnet, daß** 1/7 ≤ C/A ≤ 1/2.

10. Rauchloses Tabakprodukt nach einem der vorherigen Ansprüche, **dadurch gekennzeichnet, daß** das Vehikel ein Kompressionsmodul von weniger als 0,1 MPa hat.

11. Rauchloses Tabakprodukt nach einem der vorherigen Ansprüche, **dadurch gekennzeichnet, daß** das Vehikel eine Auflösungszeit im Bereich von 5 bis 60 Minuten, vorzugsweise im Bereich von 10 bis 30 Minuten, hat.

12. Rauchloses Tabakprodukt nach einem der vorherigen Ansprüche, **dadurch gekennzeichnet, daß** der Extrakt nikotinfrei ist.

13. Rauchloses Tabakprodukt nach einem der Ansprüche 1-11, **dadurch gekennzeichnet, daß** es sich bei dem Extrakt um Nikotin oder synthetisch hergestelltes Nikotin handelt.

## Revendications

1. Produit de tabac sans fumée sous forme d'un véhicule dissoluble (1 ; 11) destiné à l'administration par voie orale, ledit véhicule étant **caractérisé en ce que** le véhicule comprend une matrice polymère dissoluble dans laquelle de l'extrait de tabac, ou un équivalent synthétique de celui-ci, est inclus, et **en ce que** ledit véhicule dissoluble est un véhicule multicouche (11) et **en ce que** la matrice polymère dissoluble constitue au moins l'une des multiples couches (12, 13, 14).

2. Produit de tabac sans fumée selon la revendication 1, **caractérisé en ce que** l'extrait de tabac est de l'extrait de tabac séché.

3. Produit de tabac sans fumée selon la revendication 1 ou 2, **caractérisé en ce que** la couche la plus externe comprend une substance mucoadhésive.

4. Produit de tabac sans fumée selon l'une quelconque des revendications 1 à 3, **caractérisé en ce qu'**au moins une couche comprend une substance qui gonfle au contact de la salive.

5. Produit de tabac sans fumée selon l'une quelconque des revendications 1 à 4, **caractérisé en ce que** le véhicule multicouche se dissout essentiellement couche par couche dans la bouche d'un utilisateur.

6. Produit de tabac sans fumée selon l'une quelconque des revendications précédentes, **caractérisé en ce que** le véhicule a une forme essentiellement bidimensionnelle.

7. Produit de tabac sans fumée selon l'une quelconque des revendications 1 à 5, **caractérisé en ce que** le véhicule est, ou lorsqu'il est placé dans la bouche d'un utilisateur gonfle en, un véhicule tridimensionnel, qui a une première dimension A, une deuxième dimension B et une troisième dimension C, avec A ≥ B ≥ C et C/A ≥ 1/10.

8. Produit de tabac sans fumée selon la revendication 7, **caractérisé en ce que** 1/8 ≤ C/A ≤ 2/3.

9. Produit de tabac sans fumée selon la revendication 8, **caractérisé en ce que** 1/7 ≤ C/A ≤ 1/2.

10. Produit de tabac sans fumée selon l'une quelconque des revendications précédentes, **caractérisé en ce que** le véhicule a un module de compression inférieur à 0,1 MPa.

11. Produit de tabac sans fumée selon l'une quelconque des revendications précédentes, **caractérisé en ce que** ledit véhicule a un temps de dissolution dans l'intervalle de 5 à 60 minutes, de préférence dans l'intervalle de 10 à 30 minutes.

12. Produit de tabac sans fumée selon l'une quelconque des revendications précédentes, **caractérisé en ce que** ledit extrait est dépourvu de nicotine.

13. Produit de tabac sans fumée selon l'une quelconque des revendications 1 à 11, **caractérisé en ce que** ledit extrait est la nicotine ou la nicotine préparée par synthèse.
